# EUROPEAN PATENT APPLICATION

(11) **EP 1 857 110 A2**
(43) Date of publication of application: **21.11.2007**
(21) Application number: 07006725.1
(22) Date of filing: 15.03.2002
(51) Int. Cl.: A61K 31/565, A61K 31/57, A61P 5/30, A61P 5/34, A61P 15/12, A61P 15/00, A61P 15/02, A61P 19/10, A61P 9/10, A61P 3/06, A61P 39/06, A61P 35/00, A61P 25/00, A61P 11/00, A61P 17/06, A61P 31/12, A61P 7/04

(54) **Hormone replacement therapy**

(30) Priority: 16.03.2001 US 276575 P
(62) Divisional of application: 02753639.0
(71) Applicant: Wyeth, Madison, New Jersey 07940 (US)
(72) Inventor: Pickar, James, Harrison, Springfield PA 19064 (US)
(74) Representative: Mannion, Sally Kim

(57) **Abstract**

This invention relates to methods and pharmaceutical compositions for providing hormone replacement therapy in perimenopausal, menopausal, and postmenopausal women through the continuous administration of combinations of conjugated estrogens and medroxyprogesterone acetate.

## Description

### BACKGROUND

This invention relates to methods and pharmaceutical compositions for providing hormone replacement therapy in perimenopausal, menopausal, and postmenopausal women through the continuous administration of combinations of conjugated estrogens and medroxyprogesterone acetate.

Menopause is generally defined as the last natural menstrual period and is characterized by the cessation of ovarian function, leading to the substantial diminution of circulating estrogen in the bloodstream. Menopause is usually identified, in retrospect, after 12 months of amenorrhea. It is usually not a sudden event, but is often preceded by a time of irregular menstrual cycles prior to eventual cessation of menses. Following the cessation of menstruation, the decline in endogenous estrogen concentrations is typically rapid. There is a decrease in serum estrogens from circulating levels ranging from 40-250 pg/mL of estradiol and 40-170 pg/mL of estrone during ovulatory cycles to less than 15 pg/mL of estradiol and 30 pg/mL of estrone in postmenopausal women.

As these estrogens decline during the time preceding (perimenopause) and following the menopause (postmenopause), various physiological changes may result, including vulvar and vaginal atrophy causing vaginal dryness, pruritus and dyspareunia, and vasomotor instability manifested as hot flushes. Other menopausal disturbances may include depression, insomnia, and nervousness. The long-term physiologic effects of postmenopausal estrogen deprivation may result in significant morbidity and mortality due to increase in the risk factors for cardiovascular disease and osteoporosis. Menopausal changes in blood lipid levels, a major component of the pathogenesis of coronary heart disease (CHD), may be precursors to increased incidence of ischemic heart disease, atherosclerosis, and other cardiovascular disease. A rapid decrease in bone mass of both cortical (spine) and trabecular (hip) bone can be seen immediately after the menopause, with a total bone mass loss of 1% to 5% per year, continuing for 10 to 15 years.

Estrogen replacement therapy (ERT) is beneficial for symptomatic relief of hot flushes and genital atrophy and for prevention of postmenopausal osteoporosis. ERT has been recognized as an advantageous treatment for relief of vasomotor symptoms. There is no acceptable alternative to estrogen treatment for the atrophic changes in the vagina; estrogen therapy increases the vaginal mucosa and decreases vaginal dryness. Long term ERT is the key to preventing osteoporosis because it decreases bone loss, reduces spine and hip fracture, and prevents loss of height. In addition, ERT has been shown to be effective in increasing high density lipoprotein-cholesterol (HDL-C) and in reducing low density lipoprotein cholesterol (LDL-C), affording possible protection against CHD. ERT also can provide antioxidant protection against free radical mediated disorders or disease states. Estrogens have also been reported to confer neuroprotection, and inhibit neurodegenerative disorders, such as Alzheimer's disease (see U.S. Patent 5,554,601, which is hereby incorporated by reference). The following table contains a list of some of the estrogen preparations currently available in the US and Europe. Listings of such preparations are available in such as the Physicians' Desk Reference, The Orange Book, and the European equivalents thereof. Estrogen replacement therapies available in the United States and/or Europe

| Generic Name | Brand Name | Strength |
|---|---|---|
| **Oral estrogens** | | |
| Conjugated equine estrogens (natural) | Premarin | 0.3, 0.625, 0.9, 1.25, 2.5 mg |
| Conjugated estrogens (synthetic) | Cenestin | 0.625, 0.9 mg |
| Esterified estrogens (75-80% estrone sulfate, 6-15% equilin sulfate derived from plant sterols) | Estratab | 0.3, 0.625, 1.25, 2.5 mg |
| Estropipate (Piperazine estrone sulfate) | Ogen Ortho-Est | 0.625, 1.25, 2.5 mg |
| Micronized estradiol | Estrace | 0.5, 1.0, 2.0 mg |
| Raloxifene (SERM) | Evista | 60 mg |
| Esterified estrogens and methylestosterone | Estratest | 1.25 mg esterified estrogen and |
| | | 2.5 mg methylestosterone |
| | Estratest HS | 0.625 mg esterified estrogen and |
| | | 1.25 mg methylestosterone |
| Estradiol valerate | Climaval | 1 mg, 2 mg |
| Estradiol | Elleste Solo | 1 mg, 2 mg |
| Estradiol | Estrofem | 2 mg |
| Estradiol | Estrofem Forte | 4 mg |
| Piperazine esterone sulfate | Harmogen | 1.5 mg |
| Combination Estrone | Hormonin | 1.4 mg |
| Product: Estradiol | | 0.6 mg |
| Estriol | | 0.27 mg |
| Estradiol valerate | Progynova | 1 mg, 2 mg |
| Estradiol | Zumenon | 1 mg, 2 mg |

| **Transdermal estrogens** | | |
|---|---|---|
| Estradiol | Alora (twice wkly) | 0.025, 0.0375, 0.05, 0.075, |
| | Climara (weekly) | 0.1 mg of estradiol released daily (dose options for various products) |
| | Estraderm (2x wkly) | |
| | Fem Patch (wkly) | |
| | Vivelle (twice wkly) | |
| Estradiol | Dermestril | 25, 50, 100 µg |
| Estradiol | Estraderm | 25, 50, 100 µg |
| Estradiol | Evorel (Systen) | 25, 50, 75, 100 µg |
| Estradiol | Fematrix | 40, 80 µg |
| Estradiol | Menorest | 25, 37.5, 50, 75 µg |
| | Progynova TS | |
| Estradiol | And TS Forte (Climara) | 50, 100 µg |

| **Vaginal estrogens** | | |
|---|---|---|
| Conjugated equine estrogens | Premarin vaginal cream | 0.625 mg/g |
| Dienestrol | Ortho dienestrol cream | 0.1 mg/g |
| Estradiol | Estring | 7.5 µg |
| Estropipate | Ogen vaginal cream | 1.5 mg/g |
| Micronized estradiol | Estrace vaginal cream | 1.0 mg/g |

To minimize the occurrence of estrogen-related side effects and to maximize the benefit-risk ratio, the lowest dose effective in relief of symptoms and prevention of osteoporosis should be used. Although ERT reduces the relative risk (RR) for ischemic heart disease (RR, 0.50) and osteoporosis (RR, 0.40), the relative risk of endometrial cancer for postmenopausal women with a uterus may be increased. There are extensive clinical data showing that the relative risk of endometrial cancer can be reduced by the addition of a progestin, either sequentially or continuously. The addition of a progestin to estrogen therapy prevents estrogen-induced endometrial proliferation. Continuous combined hormone replacement therapy (HRT), with appropriate doses of daily estrogen and progestin, has been shown to be effective in relieving vaginal atrophy and vasomotor symptoms, preventing postmenopausal osteoporosis, and reducing the risk of endometrial cancer by prevention of endometrial hyperplasia. The following table contains a list of some currently available oral combination HRT products. Listings of such preparations are available in such as the Physicians' Desk Reference, The Orange Book, and the European equivalents thereof.

**Oral Combination HRT Products**

| Brand Name | Estrogen/Progestin | Strengths |
|---|---|---|
| Activelle | Estradiol | 1 mg |
| | Norethisterone acetate (NETA) | 0.5 mg |
| Climagest | Estradiol valerate (Climaval) | 1 or 2 mg |
| | Norethisterone (NET) | 1 mg days 17-28 |
| Cyclo Progynova | Estradiol valerate | 1 or 2 mg, days 1-21 |
| | Levonorgestrel | 250 or 500 µg, days 2-21 |
| Elleste Duet | Estradiol | 1 or 2 mg |
| | Norethisterone acetate | 1 mg days 17-28 |
| Femoston | Estradiol | 1 or 2 mg |
| | Dydrogesterone | 10 or 20 mg |
| Kliogest | Estradiol | 2 mg |
| | Norethisterone acetate | 1 mg |
| Improvera | Piperazine estrone sulfate | 1.5 mg |
| | Medroxyprogesterone acetate (MPA) | 10 mg, days 17-28 |
| Nuvelle | Estradiol valerate (Progynova) | 2 mg |
| | Levonorgestrel | 75 µg, days 17-28 |
| Premphase | Conjugated estrogens | 0.625 mg |
| | MPA | 5.0 mg |
| Prempro | Conjugated estrogens | 0.625 mg |
| | MPA | 2.5or5.0mg |
| Trisequens And Trisequens Forte | Estradiol | 2 or 4 mg, days 1-22 |
| | Norethisterone | 1 mg, days 23-28 |
| | | 1 mg, days 13-22 |
| Ortho-Prefest | Estradiol | 1.0 mg, days 1-6 |
| | Nogestimate | 0.09 mg, days 4-6 |
| Femhrt 1/5 | Ethinyl estradiol | 1.0 mg |
| | Norethindrone acetate | 5 µg |

Since it is possible that progestins ameliorate the favorable estrogen effects on lipids and may potentially impair of glucose tolerance, it is desirable, and an objective to find the lowest dose estrogen plus progestin HRT product, which also minimizes or eliminates endometrial hyperplasia. In addition, a major factor affecting a woman's decision to start and to continue taking HRT is vaginal bleeding, and many women would prefer a bleed-free product. Therefore, another objective is to provide the lowest effective dose which provides an acceptable bleeding pattern. Doses as low as NETA 0.5 mg, NET 0.35 mg, MPA 2.5 mg, levonorgesterel 0.25 mg, and dydrogesterone 5 mg have been used previously in continuous uninterrupted HRT regimens.

### DESCRIPTION OF THE INVENTION

The purpose of this invention is to provide the significant benefits of a commercially successful HRT product, such as PREMPRO (0.625 mg conjugated equine estrogens, USP plus 2.5 mg MPA), while lowering the dosage of conjugated estrogens and MPA below that which has previously been demonstrated to be effective. This invention provides a method of treating or inhibiting menopausal or postmenopausal disorders in a perimenopausal, menopausal, or postmenopausal woman in need thereof, which comprises providing to said woman, continuously and uninterruptedly over the treatment period, a combination of a daily dosage of between about 0.25 mg to about 0.1 mg conjugated estrogens (natural or synthetic) and a daily dosage of between about 2.5 mg to about 0.5 mg medroxyprogesterone acetate (MPA). The dosage is preferably provided as a pharmaceutical composition for use in treating menopausal or postmenopausal disorders which comprises a combination of conjugated estrogens and MPA. This invention further provides a pharmaceutical pack containing the daily dosage units of conjugated estrogen and MPA for continuous daily administration.

Conjugated estrogens refer to estrogenic steroidal substances in which one or more functional groups (typically hydroxyl groups) on the steroid exists as a conjugate (typically a sulfate or glucuronide). The conjugated estrogens may be a single conjugated estrogen, or may consist of mixtures of various conjugated estrogens. Numerous conjugated estrogens are described in the literature or are commercially available that are capable of being formulated for use in this invention either as a unitary estrogen, or may be mixed together with other synthetic and/or natural estrogens.

Conjugated estrogens may also contain other steroidal or non-steroidal compounds, which may, or may not, contribute to the overall biological effect. Such compounds include, but are not limited to, unconjugated estrogens, androstanes, and pregnanes. Preferred conjugated estrogens for use in this invention are PREMARIN (conjugated equine estrogens, USP, conforming with the monograph for conjugated estrogens in USP25) and CENESTIN (synthetic conjugated estrogens, A).

PREMARIN (conjugated estrogens tablets, USP) for oral administration contains a mixture of estrogens obtained exclusively from natural sources, occurring as the sodium salts of water-soluble estrogen sulfates blended to represent the average composition of material derived from pregnant mares' urine. It is a mixture of sodium estrone sulfate and sodium equilin sulfate, and at least the following 8 concomitant components, also as sodium sulfate conjugates: 17α-dihydroequilin, 17α-estradiol, Δ8,9-dehydroestrone, 17β-dihydroequilin, 17β-estradiol, equilenin, 17α-dihydroequilenin, and 17β-dihydroequilenin. PREMARIN is indicated in the treatment of moderate to severe vasomotor symptoms associated with the menopause; treatment of vulvar and vaginal atrophy; and prevention of osteoporosis, as well as other indications approved for estrogen products.

CENESTIN (synthetic conjugated estrogens, A) tablets for oral administration contain a blend of 9 synthetic estrogenic substances: sodium estrone sulfate, sodium 17α-dihydroequilin sulfate, sodium 17α-estradiol sulfate, sodium equilenin sulfate, sodium 17α-dihydroequilenin sulfate, sodium equilin sulfate, sodium 17β-dihydroequilin sulfate, sodium 17β-estradiol sulfate, sodium 17α-dihydroequilenin sulfate. CENESTIN is indicated in the treatment of moderate to severe vasomotor symptoms associated with the menopause.

PREMARIN, CENESTIN, and medroxyprogesterone acetate are all available from commercial sources (Wyeth-Ayerst - PREMARIN and medroxyprogesterone acetate; Duramed - CENESTIN).

It is preferred that the daily dosage of MPA is from about 2.5 to about 0.5 mg, with a dosage of from about 1.5 mg to about 0.5 mg being more preferred, and a dosage of from about 1 mg to about 0.5 mg being most preferred, with a daily dosage of about 1 mg per being specifically preferred. It is preferred that the conjugated estrogen constituent is PREMARIN. It is preferred that the dosage of PREMARIN is from about 0.25 mg per day to about 0.1 mg per day, and is more preferred that the dosage of PREMARIN is from about 0.2 mg per day to about 0.1 mg per day, with a daily dosage of about 0.2 mg being specifically preferred.

As used in accordance with this invention, the term "menopausal or postmenopausal disorder" refers to conditions, disorders, or disease states that are at least partially caused by the decreased estrogen production occurring during the perimenopausal, menopausal, or post-menopausal stages of a woman's life. Such disorders typically include, but are not limited to, one or more of, vaginal and vulvar atrophy, vasomotor instability, urinary incontinence, and increased risk of developing osteoporosis, cardiovascular disease, and diseases related to the oxidative damage from free radicals. As used herein, menopausal also includes conditions of decreased estrogen production that may be surgically, chemically, or be caused by a disease state which leads to premature diminution or cessation of ovarian function.

The term "daily" means that the dosage is to be administered at least once daily. The frequency is preferred to be once daily, but may be more than once daily, provided that any specified daily dosage is not exceeded.

The term "combination" of conjugated estrogens and MPA means that the daily dosage of each of the components of the combination is administered during the treatment day. The components of the combination are preferably administered at the same time; either as a unitary dosage form containing both components, or as separate dosage units; the components of the combination can be administered at different times during the day, provided that the desired daily dosage is achieved.

The term "continuous and uninterrupted" means that there is no break in the treatment regimen, during the treatment period. Thus, "continuous, uninterrupted administration" of a combination, means that the combination is administered at least once daily during the entire treatment period. It is expected that the treatment period for the combination of conjugated estrogens and MPA will be for at least 30 days, preferably 120 days, and most preferably as long term treatment, and possibly indefinite, as one of the primary reasons for administering combinations of conjugated estrogens and MPA is to treat or inhibit menopausal or postmenopausal disorders. Treatment periods also may vary depending on the symptoms to be treated. For example, for the treatment of vasomotor symptoms, it is preferred that the treatment may last from one month to several years, depending on the severity and duration of the symptoms. Physician evaluation along with patient interaction will assist the determination of the duration of treatment. For the treatment or inhibition of osteoporosis, it is preferred that the treatment period could last from six months to a number of years, or indefinitely.

This invention, also covers short term treatments or treatments of a finite term, that may be less than the 30 day preferred treatment period. It is anticipated that a patient may miss, or forget to take, one or a few dosages during the course of a treatment regimen, however, such patient is still considered to be receiving continuous, uninterrupted administration.

The term "fixed daily dosage" means that the same dosage is given every day during the treatment period. One aspect of this invention also covers situations in which a fixed daily dosage of the conjugated estrogens plus MPA combination is not given every day during the treatment period. For example, the dosage of a patient may need to be adjusted (either up or down), to achieve the desired effect during the middle of a treatment period.

The term "providing," with respect to providing a dosage of one or both of the components of this invention, means either directly administering such a component of this invention, or administering a prodrug, derivative, or analog which will form the equivalent amount of the component within the body.

It is preferred that the conjugated estrogens plus MPA combinations of this invention are provided orally. The specific dosages of conjugated estrogens plus MPA combinations of this invention that are disclosed herein are oral dosages.

In accordance with this invention, the continuously and uninterruptedly providing of a combination of a daily dosage of from about 0.25 mg to about 0.1 mg conjugated estrogens plus a daily dosage of from about 2.5 mg to about 0.5 mg of medroxyprogesterone acetate is useful in treating or inhibiting menopausal or postmenopausal disorders in perimenopausal, menopausal, or postmenopausal women. More particularly, the combinations described herein are useful in treating or inhibiting vaginal or vulvar atrophy; atrophic vaginitis; vaginal dryness; pruritus; dyspareunia; dysuria; frequent urination; urinary incontinence; urinary tract infections; vasomotor symptoms, including hot flushes, myalgia, arthralgia, insomnia, irritability, and the like; inhibiting or retarding bone demineralization; increasing bone mineral density; and treating or inhibiting osteoporosis.

The combinations of this invention also exert a cardioprotective effect in perimenopausal, menopausal, and postmenopausal women, and are therefore useful in lowering cholesterol, Lp(a), and LDL levels; inhibiting or treating hypercholesteremia; hyperlipidemia; cardiovascular disease; atherosclerosis; peripheral vascular disease; restenosis, and vasospasm; and inhibiting vascular wall damage from cellular events leading toward immune mediated vascular damage.

The combinations of this invention are antioxidants, and are therefore useful in inhibiting disorders or disease states which involve free radicals. More particularly, the combinations of this invention are useful in treating or inhibiting free radical involvement in the development of cancers, central nervous system disorders, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, amyotrophic lateral sclerosis, aging effects, adult respiratory distress syndrome, central nervous system trauma and stroke, or injury during reperfusion procedures.

The combinations of this invention are useful in treating or inhibiting dementias, neurodegenerative disorders, and Alzheimer's disease; providing neuroprotection or cognition enhancement.

The conjugated estrogens and medroxyprogesterone acetate described in this invention can be either formulated as separate tablets or as a unitary combination tablet.

Either of the components or the combination may be formulated neat or may be combined with one or more pharmaceutically acceptable carriers for administration. For example, solid carriers include starch, lactose, dicalcium phosphate, microcrystalline cellulose, sucrose and kaolin, while liquid carriers include sterile water, polyethylene glycols, non-ionic surfactants and edible oils such as corn, peanut and sesame oils, as are appropriate to the nature of the active ingredient and the particular form of administration desired. Adjuvants customarily employed in the preparation of pharmaceutical compositions may be advantageously included, such as flavoring agents, coloring agents, preserving agents, and antioxidants, for example, vitamin E, ascorbic acid, BHT and BHA.

The preferred pharmaceutical compositions from the standpoint of ease of preparation and administration are solid compositions, particularly tablets and hard-filled or liquid-filled capsules. Oral administration of the compounds is preferred.

In the Physicians' Desk Reference, PREMARIN is described as containing calcium phosphate tribasic, calcium sulfate, carnuaba wax, cellulose, glyceryl momooleate, lactose, magneseum stearate, methyl cellulose, pharmaceutical glaze, polyethylene glycol, stearic acid, sucrose, and titanium dioxide as inactive ingredients. This would be a typical formulation for PREMARIN.

CENESTIN is described as containing ethylcellulose, hydroxypropyl methylcellulose, lactose monohydrate, magnesium stearate, polyethylene glycol, polysorbate 80, pregelatinized starch, titanium dioxide, and triethyl citrate as inactive ingredients. This would be a typical formulation for CENESTIN. Formulations covering CENESTIN are described in US Patent 5,908,638, which is hereby incorporated by reference.

MPA is described as containing calcium stearate, corn starch, lactose, mineral oil, sorbic acid, sucrose, talc as inactive ingredients. This would be a typical formulaton for MPA.

Conjugated estrogens and MPA can be formulated in a number of ways to provide a single combination dosage form. Conjugated estrogens can be incorporated within the core of a compressed tablet and the progestin can be placed in an overcoating consisting of a compressed, film or sugar coat, as described in U.S. Patent 5,547,948, which is hereby incorporated by reference. The tablets described in U.S. Patent 5,547,948 are suitable for formulation of the conjugated estrogens and MPA described in this invention as a unitary tablet. U.S. Patent 5,908,638, which is hereby incorporated by reference, also describes combination tablets which are suitable for formulation of the conjugated estrogens and MPA described in this invention as a unitary tablet.

Conjugated estrogens may be formulated in a core containing the conjugated estrogens, and several components including alcohol, hydroxypropyl methyl cellulose, lactose monohydrate, magnesium stearate, and starch. The core can be covered with a coating made from components such as ethylcellulose, and triethyl citrate.

Both components can be incorporated in the compressed tablet core or in a tablet coating formulated to maintain drug stability and provide adequate oral bioavailability. For example, the progestin can be micronized.

Conjugated estrogens can be incorporated in granules, spheroids or other multiparticulate forms, and, if necessary, coated to provide adequate stability. These multiparticulates can be combined, in the appropriate proportions, with a powder blend, granulation or multiparticulates containing the progestin and incorporated into hard gelatin capsules.

Tablets of conjugated estrogens or MPA may also be cut in pieces, or crushed and placed in capsules for administration of dosages that are not specifically commercially available.

This invention also provides a pharmaceutical dose pack, containing any number of daily pharmaceutical dosage units. Preferably, and conventionally, the pack contains 28 tablets or multiples thereof. The pack should indicate that the dosage units are to be taken consecutively on a daily basis until the treatment period has ended, or until the pack has been completed. The next pack should be started on the next consecutive day. For combinations containing a unitary dosage tablet containing both conjugated estrogens and MPA, it is preferable that the pack contain one tablet corresponding to each day of administration. For combinations containing separate dosage units of conjugated estrogens and MPA, it is preferable that each one tablet of each correspond to each given day's administration, as indicated on the pill pack.

## Claims

1. Use of conjugated estrogens and medroxyprogesterone acetate in the manufacture of a pharmaceutical composition or one or more pharmaceutical dosage units, which composition or dosage unit consists of from 0.25 mg to 0.1 mg conjugated estrogens and from 2.5 mg to 0.5 mg of medroxyprogesterone acetate, and one or more pharmaceutically acceptable carriers, for the treatment of menopausal or post-menopausal disorders.

2. The use according to claim 1 wherein the conjugated estrogens is conjugated equine estrogens, USP.

3. The use according to claim 2, wherein the dosage of conjugated equine estrogens is from 0.2 mg to 0.1 mg and the dosage of medroxyprogesterone acetate is from 1.5 mg to 0.5 mg.

4. The use according to claim 3, wherein the dosage of medroxyprogesterone acetate is from 1 mg to 0.5 mg.

5. The use according to claim 4, wherein the dosage of conjugated equine estrogens, USP is 0.2 mg and the dosage of medroxyprogesterone acetate is 1 mg.

6. Use of conjugated estrogens and medroxyprogesterone acetate according to any of claims 1 to 5 for the treatment or inhibition of vasomotor symptoms in a perimenopausal, menopausal or postmenopausal woman in need thereof.

7. Use of conjugated estrogens and medroxyprogesterone acetate according to claim 6 wherein the vasomotor symptom is hot flushes.

8. Use of conjugated estrogens and medroxyprogesterone acetate according to any of claims 1 to 5 for inhibiting or retarding bone demineralization or treating or inhibiting osteoporosis in a perimenopausal, menopausal, or postmenopausal woman in need thereof.

9. Use of conjugated estrogens and medroxyprogesterone acetate according to any of claims 1 to 5 for treating or inhibiting vaginal or vulvar atrophy; atrophic vaginitis; vaginal dryness; pruritus; dyspareunia; dysuria; frequent urination; urinary incontinence; urinary tract infections in a perimenopausal, menopausal, or postmenopausal woman in need thereof.

10. Use of conjugated estrogens and medroxyprogesterone acetate according to any of claims 1 to 5 for lowering cholesterol, Lp(a), or LDL levels; inhibiting or treating hypercholesteremia; hyperlipidemia; cardiovascular disease; atherosclerosis; peripheral vascular disease; restenosis, vasospasm; or inhibiting vascular wall damage from cellular events leading toward immune mediated vascular damage, in a perimenopausal, menopausal, or postmenopausal woman in need thereof

11. Use of conjugated estrogens and medroxyprogesterone acetate according to any of claims 1 to 5 for treating or inhibiting free radical involvement in the development of cancers, central nervous system disorders, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, amyotrophic lateral sclerosis, aging effects, adult respiratory distress syndrome, central nervous system trauma and stroke, or injury during reperfusion procedures in a perimenopausal, menopausal, or postmenopausal woman in need thereof

12. Use of conjugated estrogens and medroxyprogesterone acetate according to any of claims 1 to 5 for treating or inhibiting dementias, neurodegenerative disorders, and Alzheimer's disease; providing neuroprotection or cognition enhancement in a perimenopausal, menopausal, or postmenopausal woman in need thereof

13. Use of conjugated estrogens and medroxyprogesterone acetate according to any of claims 1 to 5 for minimizing or reducing levels of breast pain in a woman receiving hormone replacement therapy.

14. Use of conjugated estrogens and medroxyprogesterone acetate according to any of claims 1 to 5 for minimizing spotting or breakthrough bleeding; or achieving amenorrhea in a woman receiving hormone replacement therapy.

15. Use of conjugated estrogens and medroxyprogesterone acetate according to claims 1 to 5 for increasing bone mineral density in a perimenopausal, menopausal, or postmenopausal woman in need thereof.
